# EUROPÄISCHE PATENTSCHRIFT

(11) **EP 1 100 367 B1**
(45) Veröffentlichungstag und Bekanntmachung des Hinweises auf die Patenterteilung: **17.05.2006**
(21) Anmeldenummer: 00943737.7
(22) Anmeldetag: 25.05.2000
(51) Int. Cl.: A61B 1/005

(54) **SCHAFT FÜR EIN FLEXIBLES ENDOSKOP**
SHAFT FOR A FLEXIBLE ENDOSCOPE
TIGE POUR ENDOSCOPE FLEXIBLE

(30) Priorität: 28.05.1999 DE 19924440
(43) Veröffentlichungstag der Anmeldung: 23.05.2001
(73) Patentinhaber: Karl Storz GmbH & Co. KG, 78532 Tuttlingen (DE)
(72) Erfinder: RENNER, Martin, D-78576 Liptingen (DE); BACHER, Uwe, D-78532 Tuttlingen (DE)
(74) Vertreter: Weller, Wolfgang
(86) Internationale Anmeldenummer: PCT/EP2000/004761
(87) Internationale Veröffentlichungsnummer: WO 2000/072743

(56) Entgegenhaltungen:
- DE-A- 3 045 237
- JP-A- 8 280 694

## Beschreibung

Die Erfindung betrifft einen Schaft für ein flexibles Endoskop, der sich von einem Kopfstück bis zu einem Endabschnitt erstreckt, mit einem schlauchförmigen Mantel, dessen außenseitige Oberfläche beim Einführen des Endoskops mit einem Körpergewebe eines Patienten in Kontakt kommt.

Darüber hinaus betrifft die Erfindung ein flexibles Endoskop mit einem derartigen Schaft.

Ein derartiges flexibles Endoskop mit einem entsprechenden Schaft ist bspw. unter der Bezeichnung Fibroskop aus dem Katalog "Endoskopie in der Tiermedizin", 5. Ausgabe, 1/95 der Karl Storz GmbH & Co., Tuttlingen, Deutschland, bekannt.

Aus der DE 30 45 237 A ist ein starres Endoskop bekannt, dessen Endoskopschaft an seiner Außenfläche mit einer oder mehreren Vertiefungen zur Aufnahme eines Gleitmittels versehen ist. Diese Vertiefung ist als eine schraubenförmige Rille ausgebildet.

Aus der JP 08 280694 A ist ein flexibles Endoskop bekannt, aus dessen distalem Ende eine flexible Sonde ausfahrbar ist. Da im Endoskop Lichtleiter zum Beleuchten des Sichtfeldes und ein Beobachtungssystem zur Rückführung des Bildes vorhanden sind und Reflexionen an der distal ausfahrbaren, krümmbaren Oberfläche der Sonde entstehen können, wird vorgeschlagen, diese aufzurauen, um Reflexionen, die das Beobachtungsbild stören, dadurch zu verhindern.

Darüber hinaus werden Fiberskope, also flexible Endoskope jedoch ebenso in der Humanmedizin vielfältig verwendet, z.B. zur Untersuchung der Atemwege.

Flexible Endoskope unterscheiden sich von starren Endoskopen dadurch, daß der Schaft bei einem flexiblen Endoskop eine solche Flexibilität aufweist, daß er eine gebogene, eine stark gekrümmte oder sogar eine schlaufenförmige Form einnehmen kann. Um die erforderliche Flexibilität zu erreichen, besteht der Schaft eines flexiblen Endoskops üblicherweise aus einem schlauchförmigen, flexiblen Mantel, in dem die für das Endoskop erforderlichen Bestandteile, wie etwa ein Bildleiter, ein Lichtleiter, ein Instrumentenkanal und Betätigungsseilzüge untergebracht sind. Der schlauchförmige Mantel besteht üblicherweise aus einem Kunststoffmaterial, das auf seiner Außenseite beispielsweise mit einer oder mehreren Schichten Polyurethan versiegelt ist.

Die Länge des Endoskopschaftes kann insbesondere bei einer Verwendung im Bereich der Tiermedizin bis hin zu 150 cm und mehr betragen. Im Bereich der Humanmedizin liegt die Länge der flexiblen Endoskopschäfte in der Regel zwischen 15 und 50 cm. Da der flexible Endoskopschaft bei der Behandlung eines Patienten oder eines Tieres über einen Großteil seiner Länge in eine häufig enge Körperhöhle vorgeschoben werden muß, ist es erforderlich, daß die außenseitige Oberfläche des Schaftes möglichst gute Gleiteigenschaften aufweist. Dies wird bei den bisher bekannten flexiblen Endoskopen dadurch erreicht, daß die Endoskope mit einer möglichst glatten Decklackschicht versehen werden, die ebenfalls aus Polyurethan bestehen kann.

In der Praxis haben sich jedoch die Gleiteigenschaften der bekannten flexiblen Endoskopschäfte in einigen Fällen, vor allem bei zunehmender Länge, als noch nicht ausreichend erwiesen. In diesen Fällen mußte dann ein zusätzliches Gleitmittel, beispielsweise in Form eines Gels, verwendet werden, um den Endoskopschaft möglichst schmerzfrei für den behandelten Patienten bzw. das behandelte Tier und möglichst einfach in die Körperhöhle vorschieben zu können. Durch die Verwendung eines Gleitmittels lassen sich die Gleiteigenschaften der Endoskopschäfte grundsätzlich verbessern. Es besteht jedoch trotzdem ein Bedürfnis, die Gleiteigenschaften der flexiblen Endoskopschäfte so optimal wie möglich zu gestalten, um die Behandlung eines Patienten oder eines Tieres so weit wie möglich zu vereinfachen und zu erleichtern. Im Optimalfall kann dann auf die Verwendung von zusätzlichen Gleitmitteln verzichtet werden.

Es ist daher Aufgabe der vorliegenden Erfindung, einen Schaft der eingangs genannten Art anzugeben, dessen Gleiteigenschaften gegenüber bisher bekannten Endoskopschäften verbessert sind. Es ist darüber hinaus Aufgabe der vorliegenden Erfindung, ein dementsprechendes flexibles Endoskop anzugeben.

Hinsichtlich des eingangs genannten Schaftes wird die genannte Aufgabe durch den Anspruch 1 dadurch gelöst, daß die außenseitige Oberfläche mikroskopisch aufgerauht ist. Ein erfindungsgemäßes flexibles Endoskop besitzt einen entsprechenden Schaft.

Mikroskopisch aufgerauht bedeutet, daß die außenseitige Oberfläche des Schaftes in einer Größenordnung aufgerauht ist, die mit bloßem Auge oder durch einfaches Anfassen nicht erkennbar ist. Gleichwohl ist die außenseitige Oberfläche des erfindungsgemäßen Schaftes gegenüber bisher bekannten Endoskopschäften gleichmäßig rauh. Ein solches Aufrauhen kann bevorzugt durch Sandstrahlen erreicht werden, was nachfolgend näher erläutert ist. Grundsätzlich ist es jedoch auch möglich, mikroskopisch rauhe Oberflächen auf andere Weise herzustellen.

In Versuchen hat sich völlig überraschend gezeigt, daß eine mikroskopisch aufgerauhte Oberfläche im Kontakt mit Körpergewebe deutlich bessere Gleiteigenschaften aufweist, als eine möglichst glatte Oberfläche, wie sie durch die bisher verwendeten, glatten Decklackschichten erreicht wurde. Eine rauhe Ausbildung der außenseitigen Oberfläche steht im krassen Gegensatz zu allen bisherigen Maßnahmen zur Verbesserung der Gleiteigenschaften, die jeweils darauf abzielten, die außenseitige Oberfläche möglichst glatt zu gestalten. Eine mögliche Erklärung für dieses überraschende Phänomen ist, daß durch das Aufrauhen eine außenseitige Oberfläche entsteht, die zahlreiche kraterförmige Vertiefungen aufweist, so daß die gesamte Kontaktfläche der außenseitigen Oberfläche mit umgebendem Körpergewebe geringer ist als im Fall von glatten außenseitigen Oberflächen. Unabhängig von diesem Versuch einer Erklärung bietet die genannte Maßnahme jedoch eine sehr einfache und gleichzeitig ausgesprochen wirkungsvolle Möglichkeit, die Gleiteigenschaften von flexiblen Endoskopschäften zu verbessern.

Insgesamt ist die gestellte Aufgabe daher vollständig gelöst.

In einer Ausgestaltung der zuvor genannten Maßnahme ist die außenseitige Oberfläche durch Sandstrahlen aufgerauht.

Diese Maßnahme besitzt den Vorteil, daß das Aufrauhen der außenseitigen Oberfläche hierbei durch eine einfache und mit herkömmlichen Technologien beherrschbare Maßnahme erfolgt. Diese Maßnahme kann sich als letzter Fertigungsschritt bei der Herstellung des Schaftes ohne wesentlichen Aufwand an die bereits bisher durchgeführten Fertigungsschritte anschließen. Die genannte Maßnahme besitzt somit den Vorteil, daß die erfindungsgemäße Verbesserung der Gleiteigenschaften vor allem in fertigungstechnischer Hinsicht besonders einfach und kostengünstig ist. Darüber hinaus besitzt die Maßnahme den Vorteil, daß hierdurch auch bereits herkömmlich hergestellte Endoskopschäfte nachbehandelt werden können, so daß auch eine nachträgliche Verbesserung der Gleiteigenschaften von bereits hergestellten oder sogar bereits verwendeten Endoskopschäften möglich ist.

In einer weiteren Ausgestaltung der zuvorgenannten Maßnahme ist die außenseitige Oberfläche durch Sandstrahlen mit kantigen Körnern aufgerauht.

Grundsätzlich ist es auch bekannt, Oberflächen durch Sandstrahlen mit kugelförmigen, also nicht-kantigen Körnern aufzurauhen. Versuche haben jedoch gezeigt, daß die Verbesserung der Gleiteigenschaften bei der Verwendung von kantigen Körnern besonders ausgeprägt ist und besonders schnell erreicht wird.

In einer weiteren Ausgestaltung der Erfindung ist die außenseitige Oberfläche in einer Rauheit aufgerauht, die durch Sandstrahlen mit Korund 0,05 - 0,5 mm erreichbar ist.

Die Zahlenangabe 0,05 - 0,5 mm gibt dabei in an sich bekannter Weise die Korngröße der zum Sandstrahlen verwendeten Korundkörner an. Eine Verwendung anderer Korngrößen oder auch anderer Schleifmaterialien als Korund wird bei dieser Ausgestaltung der Erfindung jedoch nicht generell ausgeschlossen. Entscheidend ist hier, daß eine Rauheit der außenseitigen Oberfläche erreicht wird, die derjenigen entspricht, die mit Hilfe der genannten Maßnahme erreichbar ist. Es hat sich nämlich gezeigt, daß eine derartige Rauheit einerseits eine beträchtliche Verbesserung der Gleiteigenschaften gegenüber bisher bekannten Endoskopschäften bedeutet, während andererseits die außenseitige Oberfläche der Endoskopschäfte so keine spürbaren Degradationen im Hinblick auf eine Sterilisierbarkeit und eine damit verbundene wiederholte Verwendung bei der Behandlung von Patienten oder Tieren erfährt. Die genannte Maßnahme ist das Ergebnis empirischer Versuche und stellt eine hervorragende Verbesserung der Gleiteigenschaften gegenüber bisher verwendeten flexiblen Endoskopschäften dar. Ebenfalls sehr gute Ergebnisse konnten durch Sandstrahlen mit Glasperlen 40 - 70 µm bzw. 70 - 110 µm erreicht werden.

In einer weiteren Ausgestaltung ist die außenseitige Oberfläche durch Ätzen aufgerauht.

Diese Maßnahme besitzt den Vorteil, daß das Aufrauhen auf sehr einfache Weise besonders gleichmäßig geschehen kann. Im einfachsten Fall wird einfach der vorher glatte Endoskopschaft eine bestimmte Zeit lang in eine geeignetes Ätzbad gelegt. Dabei wird die außenseitige Oberfläche chemisch aufgerauht. Das erforderliche Ätzmaterial hängt von dem Material ab, das zur Bildung der außenseitigen Oberfläche verwendet wurde. Geeignete Ätzmaterialien sind im Stand der Technik an sich bekannt.

In einer weiteren Ausgestaltung ist die außenseitige Oberfläche durch Schleifen aufgerauht.

Unter Schleifen wird dabei im Unterschied zum Sandstrahlen ein mechanisches Aufrauhen der außenseitigen Oberfläche verstanden, das mit Hilfe einer Schleifscheibe, mit Hilfe von Schleifpapier oder mit Hilfe von ähnlichen körperlich gebundenen Schleifkörnern vorgenommen wird. Eine derartige Maßnahme besitzt den Vorteil, daß hierbei das Aufrauhen örtlich sehr gezielt und differenziert erfolgen kann, wodurch verschiedene Stellen des Endoskopschaftes gezielt unterschiedlich stark aufgerauht werden können. Dabei können für verschiedene Bereiche des Schaftes beispielsweise unterschiedliche Körnungen eingesetzt werden, um stellenweise unterschiedliche Rauheit und damit unterschiedliche Gleiteigenschaften zu erhalten.

In einer weiteren Ausgestaltung der Erfindung besteht die außenseitige Oberfläche aus einem an sich glänzenden Material und sie ist so weit aufgerauht, daß sie matt erscheint.

Auch diese Maßnahme ist das Ergebnis empirischer Versuche. Zugrunde gelegt wurden hierbei schlauchförmige Mäntel für Endoskopschäfte, wie sie aus dem eingangs genannten Katalog der Firma Karl Storz GmbH & Co., Tuttlingen, Deutschland bekannt sind. Bei diesen bekannten Endoskopschäften ist die außenseitige Oberfläche durch einen transparenten, nach dem Aushärten glänzend erscheinenden Decklack aus Polyurethan gebildet. Zur Verbesserung der Gleiteigenschaften wird diese außenseitige Oberfläche vorzugsweise so weit aufgerauht, daß sie matt erscheint. Die genannte Maßnahme besitzt den Vorteil, daß sie ein einfaches und leicht überprüfbares Kriterium bei der Fertigung der erfindungsgemäßen Endoskopschäfte darstellt. Die Herstellung der erfindunggemäßen Endoskopschäfte ist somit ohne größeren Eingriff in die bereits bisher durchgeführten Fertigungsschritte möglich. Insgesamt lassen sich aufgrund der genannten Maßnahme auf einfache Weise beträchtliche Verbesserungen der Gleiteigenschaften erreichen.

In einer weiteren Ausgestaltung der Erfindung weist der schlauchförmige Mantel zumindest eine Basisschicht und eine darüber angeordnete Deckschicht auf, wobei nur die Deckschicht aufgerauht ist.

Die genannte Maßnahme besitzt den Vorteil, daß hierbei eine Verletzung der inneren Mantelbereiche der erfindungsgemäßen Endoskopschäfte vermieden ist. Hierdurch ist gewährleistet, daß trotz der erfindungsgemäßen Maßnahme auch ein häufiges Sterilisieren die innerhalb der Endoskopschäfte angeordneten Bestandteile des Endoskops nicht beeinträchtigt.

Nach einer weiteren Ausgestaltung der Erfindung liegt der Mittenrauhwert Rₐ der aufgerauhten Oberfläche im Bereich von 0,1 bis 1,6 µm.

Diese Maßnahme hat den Vorteil, daß Oberflächen mit diesen Mittenrauhwerten besonderes gute Gleiteigenschaften entlang von Gewebe zeigen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine perspektivische Ansicht eines erfindungsgemäßen flexiblen Endoskops,
- Fig. 2: einen Schnitt durch den Schaft des erfindungsgemäßen Endoskops aus Fig. 1 entlang der Linie II-II, und
- Fig. 3: eine stark vergrößerte Darstellung des schlauchförmigen Mantels des flexiblen Endoskops aus Fig. 2.

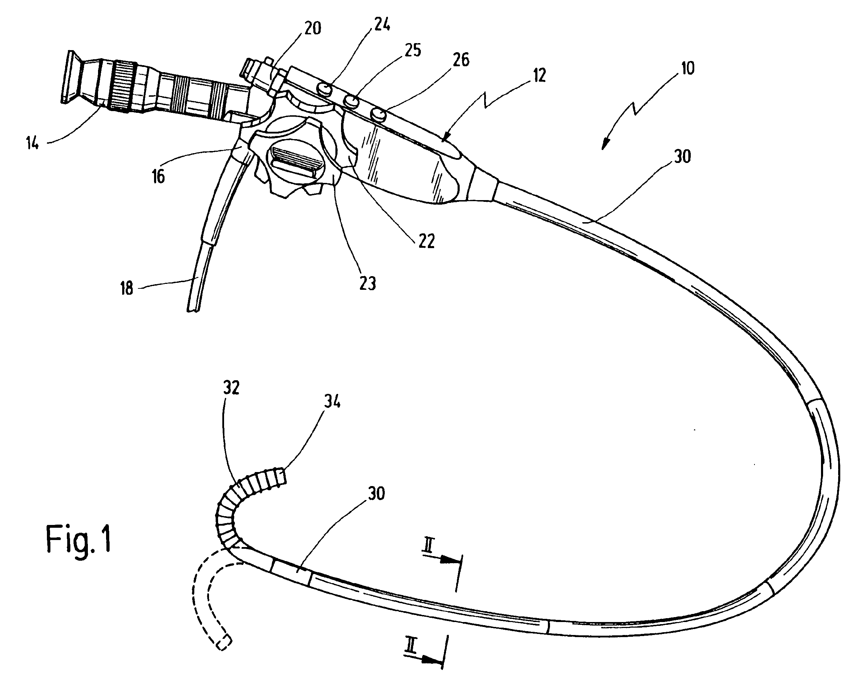

In Fig. 1 ist ein erfindungsgemäßes flexibles Endoskop in seiner Gesamtheit mit der Bezugsziffer 10 bezeichnet.

Das flexible Endoskop 10 weist ein insgesamt mit der Bezugsziffer 12 bezeichnetes Kopfstück auf. Proximalseitig steht vom Kopfstück 12 ein Okular 14 vor. Ein seitlich vorstehender Anschluß 16 dient dazu, einen Leitungsstrang 18 anzuschließen, der Leitungen für Beleuchtung, Spülung, Insufflation, Absaugung und dergleichen enthalten kann. Ein weiterer, sich etwa in Richtung des Okulars 14 erstreckender Anschluß 20 ist dazu vorgesehen, daß über diesen Instrumente, beispielsweise Zangen, Schlingen oder dergleichen, in das Endoskop 10 bzw. das Kopfstück 12 eingeschoben werden können.

Seitlich am Kopfstück 12 sind zwei Handräder 22, 23 angeordnet, mit denen die Blickrichtung des Endoskops, wie nachfolgend noch erläutert, verstellt werden kann. Im Bereich des Kopfstücks 12 sind ferner Schalter 24, 25, 26 angeordnet, über die verschiedene Funktionen, wie beispielsweise Saugen, Spülen oder dergleichen, gesteuert werden können, was an sich bei der Ausgestaltung von flexiblen Endoskopen bekannt ist.

Vom Kopfstück 12 erstreckt sich ein langgestreckter flexibler Schaft 30 fort, der im dargestellten Ausführungsbeispiel die Länge von etwa 1 m aufweist. Der Schaft 30 ist aus einem flexiblen Kunststoffmaterial hergestellt, das ein Krümmen und Biegen des Schaftes 30, wie in Fig. 1 dargestellt, ermöglicht. Ein Endabschnitt 32 weist gegenüber dem Schaft 30 eine nochmals erhöhte Biegsamkeit dahingehend auf, daß der Endabschnitt 32 zusätzlich noch um mehr als 180° halbkreisförmig abgebogen werden kann, wie das aus Fig. 1 ersichtlich ist. Über einen Zugseilmechanismus, der sich von einem Abschlußstück 34 bis zu den Handrädern 22, 23 erstreckt, kann der Endabschnitt 32 bei an sich unveränderter Orientierung des Schaftes 30 aus der in Fig. 1 in durchgezogenen Linien dargestellten Position um mehr als 180° verschwenkt werden. Eine verschwenkte Position des Endabschnitts 32 ist in Fig. 1 mit unterbrochenen Linien dargestellt. Die auf diese Weise erreichte Beweglichkeit des Endabschnitts 32 ist bei flexiblen Endoskopen an sich bekannt und ermöglicht eine Verstellung der Blickrichtung des Endoskops.

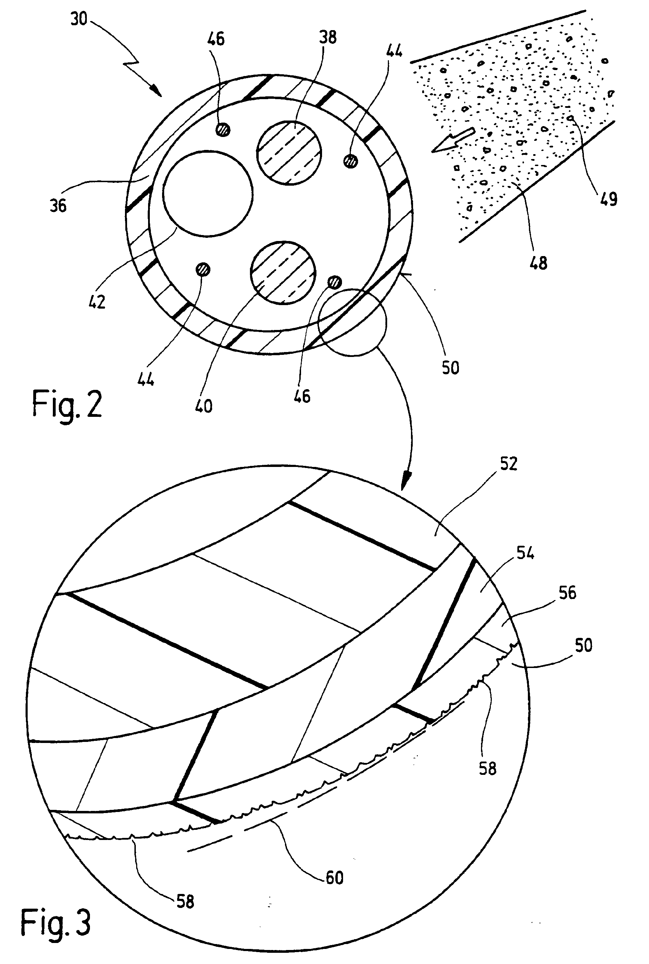

In der Querschnittsdarstellung von Fig. 2 ist der an sich ebenfalls bekannte Innenaufbau des Schaftes 30 des Endoskops 10 erkennbar. Der Schaft 30 besitzt einen schlauchförmigen Mantel 36, in dessen Innerem ein Bildleiter 38, ein Lichtleiter 40, ein Instrumentenkanal 42 sowie Seilzüge 44, 46 verlaufen. Die Seilzüge 44, 46 sind mit den Handrädern 22, 23 verbunden und ermöglichen die bereits beschriebene Bewegung des Endstücks 32.

Mit der Bezugsziffer 48 ist schematisch ein Sandstrahlen der außenseitigen Oberfläche 50 angedeutet. Durch das Sandstrahlen 48 wird die außenseitige Oberfläche 50 mikroskopisch aufgerauht. Hierdurch lassen sich die Gleiteigenschaften der außenseitigen Oberfläche 50 des Mantels 36 und damit die Gleiteigenschaften des Schaftes 30 verbessern. Bevorzugt erfolgt das Sandstrahlen 48 zu diesem Zweck mit kantigen Körnern 49.

In einem besonders bevorzugten Ausführungsbeispiel ist die außenseitige Oberfläche 50 des Mantels 36 durch Sandstrahlen 48 mit Korund 0,12 - 0,25 mm aufgerauht. Die Zahlenangaben 0,12 - 0,25 mm beziehen sich dabei, wie bereits erwähnt, auf die Korngröße der verwendeten Körner 49. Derartige Größenbezeichnungen sind im Bereich des Sandstrahlens hinlänglich bekannt. In einem alternativen Ausführungsbeispiel ist die außenseitige Oberfläche 50 des Mantels 36 durch Sandstrahlen mit Glasperlen 40 - 70 µm bzw. 70 - 110 µm aufgerauht.

Entgegen der in dieser Hinsicht schematischen Darstellung in Fig. 2 erfolgt das Sandstrahlen 48 der außenseitigen Oberfläche 50 bevorzugt zu einem Zeitpunkt, zu dem der Bildleiter 38, der Lichtleiter 40, der Instrumentenkanal 42 und die Seilzüge 44, 46 noch nicht in den Schaft 30 eingebaut sind.

In alternativen Ausführungsbeispielen der Erfindung ist die außenseitige Oberfläche 50 durch Ätzen in einem geeigneten Ätzbad oder durch Schleifen mit einem Schleifkörper wie etwa einer Schleifscheibe aufgerauht.

In der nachfolgenden Tabelle 1 sind die Rauhigkeitscharakteristika (nach DIN 4768 T1; 8.74) von aufgerauhten Schäften von Fiberskopen der Anmelderin aufgelistet, die mit unterschiedlichen Aufrauhmitteln aufgerauht worden sind.

**Tabelle 1 Rauhigkeitscharakteristika (DIN 4768 Tl; 8.74)**

| | Schaft 1 | Schaft 2 | Schaft 3 |
|---|---|---|---|
| Rₐ | 0,42 µm | 0,70 µm | 0,90 µm |
| R_{z} | 2,5 µm | 4,20 µm | 5,30 µm |
| Rₘₐₓ | 3,3 µm | 5,2 µm | 6,5 µm |

| | | | |
|---|---|---|---|
| Rₐ = Mittenrauhwert | | | |
| R_{z} = Rauhtiefe | | | |
| Rₘₐₓ = maximale Rauhtiefe | | | |

In der vergrößerten Darstellung der Fig. 3 ist erkennbar, daß die außenseitige Oberfläche 50 des Mantels 36 durch das Sandstrahlen 48 aufgerauht ist. Darüber hinaus ist in dieser vergrößerten Ansicht dargestellt, daß der Mantel 36 eine erste Basisschicht 52 aufweist, die aus einem flexiblen Kunststoffmaterial besteht. Diese erste Basisschicht 52 bildet den tragfähigen Innenbereich des Mantels 36. Außenseitig ist die erste Basisschicht 52 durch eine zweite Basisschicht 54 versiegelt. Die zweite Basisschicht 54 besteht im vorliegenden Ausführungsbeispiel aus einem weich eingestellten Polyurethan. In einem bevorzugten Ausführungsbeispiel, das hier aus Gründen der Übersichtlichkeit nicht gezeichnet ist, besteht die zweite Basisschicht 54 aus drei einzelnen Polyurethanschichten, die von der Innenseite des Mantels 36 nach außen eine abnehmende Härte aufweisen. Auf die äußerste Basisschicht 54 ist außenseitig eine Deckschicht 56 aufgetragen. Die Deckschicht 56 besteht aus einem transparenten Decklack, der nach dem Aushärten zunächst eine sehr glatte, glänzende Oberfläche 50 bildet. Diese Oberfläche 50 wird anschließend durch das Sandstrahlen 48 oder durch die alternative genannten Möglichkeiten in der in Fig. 2 und 3 dargestellten Weise mikroskopisch aufgerauht.

In einem bevorzugten Ausführungsbeispiel der Erfindung wird die außenseitige Oberfläche 50 des Mantels 36 so weit aufgerauht, daß die außenseitige Oberfläche 50 nur noch matt erscheint. Im mikroskopischen Bereich führt dies dazu, daß die außenseitige Oberfläche 50 kraterförmige Vertiefungen 58 erhält.

Mit der Bezugsziffer 60 ist schematisch die Oberfläche eines Körpergewebes angedeutet, das bei der Verwendung des erfindungsgemäßen Endoskops 10 mit der außenseitigen Oberfläche 50 in Berührung kommt. Wie dargestellt, hat das Aufrauhen der außenseitigen Oberfläche 50 zur Folge, daß das Körpergewebe 60 nicht mehr mit den kraterförmigen Vertiefungen 58 in Kontakt kommt, sondern nur noch mit den dazwischen verbliebenen Plateaus. Hierdurch ist die gesamte Kontaktfläche gegenüber den Verhältnissen bei den bisher bekannten Endoskopschäften 30 verringert. Infolgedessen besitzt der Schaft 30 des erfindungsgemäßen Endoskops 10 verbesserte Gleiteigenschaften.

Der Druck und die Dauer, mit der die außenseitige Oberfläche 50 des Mantels 36 beim Sandstrahlen 48 beaufschlagt wird, wird bevorzugt empirisch bestimmt, und zwar danach, daß die vormals glänzende Oberfläche 50 der Deckschicht 56 nach dem Sandstrahlen 48 gleichmäßig matt erscheint.

## Patentansprüche

1. Schaft (30) für ein flexibles Endoskop, wobei der Schaft einen schlauchförmigen Mantel (36) aufweist, dessen außenseitige Oberfläche (50) beim Gebrauch des Endoskops (10) mit einem Körpergewebe (60) eines Patienten in Kontakt kommt, **dadurch gekennzeichnet, daß** die außenseitige Oberfläche (50) mikroskopisch aufgerauht ist.

2. Schaft nach Anspruch 1, **dadurch gekennzeichnet, daß** die außenseitige Oberfläche (50) durch Sandstrahlen (48) aufgerauht ist.

3. Schaft nach Anspruch 2, **dadurch gekennzeichnet, daß** die außenseitige Oberfläche (50) durch Sandstrahlen (48) mit kantigen Körnern (49) aufgerauht ist.

4. Schaft nach einem der Ansprüche 1 bis 3, **dadurch gekennzeichnet, daß** die außenseitige Oberfläche (50) in einer Rauheit aufgerauht ist, die durch Sandstrahlen (48) mit Korund 0,05 - 0,5 mm erreichbar ist.

5. Schaft nach Anspruch 1, **dadurch gekennzeichnet, daß** die außenseitige Oberfläche (50) durch Ätzen aufgerauht ist.

6. Schaft nach Anspruch 1, **dadurch gekennzeichnet, daß** die außenseitige Oberfläche (50) durch Schleifen aufgerauht ist.

7. Schaft nach einem der Ansprüche 1 bis 6, **dadurch gekennzeichnet, daß** die außenseitige Oberfläche (50) aus einem an sich glänzenden Material besteht und daß die außenseitige Oberfläche (50) soweit aufgerauht ist, daß sie matt erscheint.

8. Schaft nach einem der Ansprüche 1 bis 7, **dadurch gekennzeichnet, daß** der schlauchförmige Mantel (36) zumindest eine Basisschicht (52, 54) und eine darüber angeordnete Deckschicht (56) aufweist, wobei nur die Deckschicht (56) aufgerauht ist.

9. Schaft nach einem der Ansprüche 1 bis 8, **dadurch gekennzeichnet, daß** der Mittenrauhwert Rₐ der aufgerauhten Oberfläche (50) im Bereich von 0,1 µm bis 1,6 µm liegt.

10. Flexibles Endoskop mit einem Schaft nach einem der Ansprüche 1 bis 9.

## Claims

1. Shaft (30) for a flexible endoscope, wherein the shaft has a tubular mantle (36) whose outer surface (50) contacts a body tissue (60) of a patient during use of the endoscope (10), **characterized in that** the outer surface (50) is microscopically roughened.

2. Shaft of claim 1, **characterized in that** the outer surface (50) is roughened by a sand blasting (48).

3. Shaft of claim 1, **characterized in that** the outer surface (50) is roughened by sand blasting (48) with grains (49) having edges.

4. Shaft of anyone of claims 1 through 3, **characterized in that** the outer surface (50) is roughened to a roughness which is achievable by sand blasting (48) with corundum 0.05 to 0.5 mm.

5. Shaft of claim 1, **characterized in that** the outer surface (50) is roughened by etching.

6. Shaft of claim 1, **characterized in that** the outer surface (50) is roughened by grinding.

7. Shaft of anyone of claims 1 through 6, **characterized in that** the outer surface (50) consists of a material that is initially glossy and **in that** the outer surface (50) is roughened to an extent that it appears dull.

8. Shaft of anyone of claims 1 through 7, **characterized in that** the tubular mantle (36) comprises at least one base layer (52, 54) and a cover layer (56) laying thereon, wherein only the cover layer (56) is roughened.

9. Shaft of anyone of claims 1 through 8, **characterized in that** the average roughness value Rₐ of the roughened surface (50) lies in the range of 0.1 µm to 1.6 µm.

10. Flexible endoscope with a shaft according to anyone of claims 1 through 9.

## Revendications

1. Tige (30) pour un endoscope flexible, la tige présentant une enveloppe (36) en forme de gaine dont la surface (50) côté extérieur vient en contact avec un tissu corporel (60) d'un patient, pendant l'utilisation de l'endoscope (10), **caractérisée en ce que** la surface (50) côté extérieur présente une rugosité microscopique.

2. Tige selon la revendication 1, **caractérisée en ce que** la surface (50) côté extérieur est rendue rugueuse par sablage (48).

3. Tige selon la revendication 2, **caractérisée en ce que** la surface (50) côté extérieur est rendue rugueuse par sablage (48) avec des grains (49) à arêtes vives.

4. Tige selon l'une des revendications 1 à 3, **caractérisée en ce que** la surface (50) côté extérieur est rendue rugueuse avec une rugosité qui peut être obtenue par sablage (48) avec un corindon de 0,05 à 0,5 mm.

5. Tige selon la revendication 1, **caractérisée en ce que** la surface (50) côté extérieur est rendue rugueuse par décapage.

6. Tige selon la revendication 1, **caractérisée en ce que** la surface (50) côté extérieur est rendue rugueuse par meulage.

7. Tige selon l'une des revendications 1 à 6, **caractérisée en ce que** la surface (50) côté extérieur est constituée d'un matériau brillant et **en ce que** la surface (50) côté extérieur est rendue rugueuse jusqu'à ce qu'elle apparaisse mate.

8. Tige selon l'une des revendications 1 à 7, **caractérisée en ce que** l'enveloppe (36) en forme de gaine comporte au moins une couche de base (52, 54) et une couche de couverture (56) disposée au-dessus, seule la couche de couverture (56) étant rendue rugueuse.

9. Tige selon l'une des revendications 1 à 8, **caractérisée en ce que** la valeur moyenne de rugosité Rₐ de la surface (50) rendue rugueuse se situe entre 0,1 µm et 1,6 µm.

10. Endoscope flexible avec une tige selon l'une des revendications 1 à 9.
